# EUROPEAN PATENT SPECIFICATION

(11) **EP 2 687 331 B1**
(45) Date of publication and mention of the grant of the patent: **04.03.2015**
(21) Application number: 12177057.2
(22) Date of filing: 19.07.2012
(51) Int. Cl.: B24B 55/00, A61B 5/00, B23Q 11/00, F16P 3/00, G01H 1/00, A47L 7/00

(54) **A vacuum cleaner connected to a hand held or hand guided tool and comprising a vibration monitoring device**
Staubsauger, der an ein tragbares oder handgesteuertes Werkzeug mit einer Schwingungsüberwachungsvorrichtung angeschlossen ist.
Un aspirateur relié à un outil portable ou guidé à la main comprenant un dispositif de surveillance de vibrations

(43) Date of publication of application: 22.01.2014
(73) Proprietor: Valentini, Guido, 20122 Milano (IT)
(72) Inventor: Valentini, Guido, 20122 Milano (IT)
(74) Representative: DREISS Patentanwälte PartG mbB

(56) References cited:
- EP-A2- 2 047 784
- WO-A2-2007/072068
- GB-A- 2 435 001
- JP-A- 2006 068 324

## Description

The present invention refers to a vacuum cleaner adapted for connection to a hand held or hand guided tool generating vibrations during its operation and transmitting these to an operator using the tool and for aspiring dust generated during operation of the tool. The vacuum cleaner is automatically activated upon activation of the tool and automatically deactivated after deactivation of the tool by means of an electrical signal.

Separate vibration monitoring devices for hand held or hand guided power tools are known, for example, from GB 2 435 001 A and WO 2007/072068 A2. EP 2 047 784 A2 discloses a vacuum cleaner adapted for connection to a hand held or hand guided power tool. The known vacuum cleaner comprises a power sensing circuit which enable automatic activation of the vacuum cleaner upon activation of the power tool. Finally, JP 2006 068324 A describes a vacuum cleaner comprising an integral device for calculating and displaying calories consumed by the user of the vacuum cleaner during the cleaning process.

The problem of excessive vibrations caused by a hand held or hand guided tool and transmitted to an operator using the tool is a problem well known in the art. This problem is also addressed in various official standards, for example in European Standard EN 60745 defining certain maximum cumulative vibration values for electric power tools and/or EN 792-8, EN 28662 and EN 28927 for pneumatic power tools. The cumulative vibrations an operator, who uses the tool for a certain period of time, is exposed to must not exceed the maximum vibration values defined in the standard.

The cumulative vibration level the user of a tool is exposed to depends on a vibration value and on the duration of exposure. The vibration value may be a predefined vibration value characteristic determined by the manufacturer of a tool for that tool before use of the tool and usually quoted in the tool's user manual or data sheet as one of many characteristic parameters of the tool. However, the vibration value characteristic of each tool may vary during use of the tool, for example due to a change or shifting of the tool's vibrating masses. Originally, the current cumulative vibration level the operator using the vibrating tool has been exposed to so far during the current use of the tool has simply been estimated by the operator. For this purpose the vibration value characteristic for the tool predefined by the manufacturer of the tool was considered and the duration of use of the tool was roughly estimated, for example by manually starting a timer when the operator began using the tool and manually stopping the timer when he had definitely finished using the tool. However, between manually starting and stopping the timer the operator usually does not use the tool without interruption. Rather, vibrating tools are usually used intermittently. Hence, during the period of use between starting and stopping the timer the tool is often removed from the work piece thereby significantly reducing the effect if vibrations on the user or the tool is temporarily turned off completely, for example for inspecting the work piece and/or the work piece surface treated so far. Consequently, in the prior art estimating the overall cumulative vibration level the operator is exposed to during use of the tool is rather inaccurate and error-prone.

In order to be able to determine the cumulative vibration level the operator is exposed to during use of the tool more precisely, it is suggested in WO 2007/072068 A2 to make use of a hand held vibration monitoring apparatus. The monitoring apparatus comprises a vibration sensor and a timer. The vibration sensor is adapted to measure the current vibrations of the hand held tool. If the vibrations exceed a defined value, they are stored for calculation of the overall vibration level the operator is exposed to. The timer is automatically started if the vibrations measured by the vibration sensor exceed a defined value and automatically stopped it the measured vibrations fall below the defined value. The overall cumulative vibration level the operator is exposed to during use of the tool is calculated based on the measured vibrations and on the duration of use provided by the timer. Hence, it can be exactly determined whether the maximum allowed cumulative vibration level has been reached or even exceeded.

However, it is a problem of the known prior art vibration monitoring apparatus that the timer is started and stopped depending on the current vibration values measured by the vibration sensor. Hence, the known monitoring apparatus has to be provided with a vibration sensor for measuring the current vibrations and/or for starting and stopping the timer. This makes the monitoring apparatus rather large, heavy, complex and expensive. Furthermore, the monitoring apparatus with the vibration sensor has to be rigidly fastened to the tool or directly at the operator's hand, in order to safely and reliably measure the actual vibrations. This strongly limits the possible embodiments of the vibration monitoring apparatus and makes it impossible to provide a small and light weight vibration monitoring apparatus detachable from the tool and/or the operator's hand.

It is an object of the present invention to provide the user of a hand held or hand guided tool with information regarding the tool's vibration and/or the remaining possible time of use without the necessity of a separate apparatus for measuring and displaying the information.

This object is solved by a vacuum cleaner, which is adapted for connection to a hand held or hand guided tool generating vibrations during its operation and transmitting these to an operator using the tool according to the preamble of claim 1. According to an aspect of the present invention, the vacuum cleaner comprises a monitoring device for calculating the overall vibration level the operator is exposed to during use of the tool, the calculation depending on a vibration value indicative of the quantity of vibrations generated by the tool during its operation and on an exposure time value indicative of the duration the operator is exposed to the vibrations.

Vacuum cleaners according to the present invention are connected to a hand held or hand guided tool in order to aspire dust generated during operation of the tool. According to the present invention the vacuum cleaner is equipped with the monitoring device for calculating the overall vibration level the operator is exposed to during use of the tool. Hence, instead of equipping the tool with the monitoring device thereby requiring additional space within the tool and significantly enhancing the tool's size and weight, the monitoring device is separated from the tool and located in a vacuum cleaner. This has the advantage that the size and the weight of the tool are not enhanced by the monitoring device. Further, locating the monitoring device in the vacuum cleaner has the advantage that information regarding the vibrations, in particular the overall vibration level the operator is exposed to, the time the operator has been exposed to the vibrations and/or the remaining time the operator may still use the tool without exceeding the predefined permissible maximum accumulated vibration level can be output to the operator and the operator can easily and conveniently comprehend the information displayed to him because the vacuum cleaner is usually located near the operator using the tool. Besides - in contrast to hand held or hand guided tools - a vacuum cleaner usually has enough free surface on its outer casing in order to provide a large and clearly visible display.

The idea of integrating a vibration monitoring device into a vacuum cleaner is not at all straight forward because the manufacturer of a hand held or hand guided power tool is different from the manufacturer of a vacuum cleaner. Usually, the tool manufacturers buy conventional vacuum cleaners from the vacuum cleaner manufacturers and resell them to their own clients for use together with power tools. The power tools are mainly made for professional use, whereas the vacuum cleaners are originally made for domestic use. Up to now it has never been thought of integrating some kind of power tool function into a vacuum cleaner adapted for use together with the tool because there was hardly any communication and co-operation between the manufacturers of power tools and vacuum cleaners on the level of developing vacuum-cleaners. It has never been thought of integrating the controls for certain power tool functions into a vacuum cleaner and/or of integrating a display in the vacuum cleaner's casing for the display of one or more power tool values (speed, applied force, etc.) and/or the current operational status of the power tool.

In the present case the integration of the monitoring device into the vacuum cleaner is particularly advantageous if the vibration value assigned to the tool is a pre-set value which can be provided directly to the monitoring device prior to the calculation of the overall vibration level. Hence, there is no need for a continuous data transmission from a vibration sensing device located somewhere near or inside the power tool to the vibration monitoring device located in the vacuum cleaner during runtime of the power tool. The signal for automatically starting the timer when power and for automatically stopping the timer after interrupting the power supply is already present in the vacuum cleaner anyway.

According to a preferred embodiment of the present invention the exposure time value is derived from a timer, which is automatically started and/or stopped according to the electrical signal for activating and/or deactivating the vacuum cleaner depending on the activation and/or deactivation of the tool. This embodiment has the advantage that the timer for determining the exposure time value is started and/or stopped by means of an electrical signal which is present in the tool and/or the vacuum cleaner anyway. No additional signal for starting and/or stopping the timer and no additional means for generating that signal are necessary. The electrical signal for starting and/or stopping the timer is derived form a measure directly indicative of activation and/or deactivation of the tool independent from the actual value of the vibrations currently generated by the tool and to which the operator is currently exposed.

The electrical signal for automatically activating the vacuum cleaner upon activation of the tool preferably activates the vacuum cleaner simultaneously to the activation of the tool. The deactivation of the vacuum cleaner by means of the electrical signal can be effected synchronously to the deactivation of the tool. However, it is also possible that the electrical signal provides for a delayed deactivation of the vacuum cleaner, that is only after a certain period of time after the tool's deactivation. This means that after deactivation of the tool the vacuum cleaner continues to work for a certain period of time in order to aspire remaining dust from the previous working session. This can be effected in that the electrical signal provoking the deactivation of the vacuum cleaner assumes its deactivation value only after the certain period of time after the deactivation of the tool. Alternatively, the electrical signal assumes its deactivation value synchronously with the deactivation of the tool and the deactivation value of the electrical signal has the deactivation effect on the vacuum cleaner only with a delay corresponding to the certain period of time. For that purpose appropriate delay means can be integrated in the vacuum cleaner.

According to another preferred embodiment of the present invention the monitoring device is integrally formed with a casing of the vacuum cleaner. Of course not the entire monitoring device has to be integrated in the casing. It would be sufficient to integrate the monitoring device's display and - if present - some actuating and/or inserting means (for example push-buttons, an actuator device, etc.) in the casing in order to provide an interface for communication with the operator. The processing unit and other components of the vibration monitoring device, such as the timer, could be provided at some other point in the vacuum cleaner separate and possibly distant from the casing. The monitoring device and the timer can be embodied as separate units. However, preferably the timer is an integral part of the monitoring device. This embodiment has the advantage that no additional unit separate from the tool and the vacuum cleaner has to be handled during the use of the tool and the vacuum cleaner. Further, with the monitoring device or rather its display being integrally formed within the casing of the vacuum cleaner it is possible for the operator of the tool to easily acquire the current status of the operator's vibration exposure, because the vacuum cleaner is usually located within the operator's range of sight when using.the tool. The operator can acquire the information regarding the cumulative vibration exposure level and/or the exposure time value and/or other information relating to his exposure to vibrations even during continuous use of the tool. A relatively large display unit located in the casing of the vacuum cleaner can provide the operator clearly and unambiguously with the desired information regarding the current vibration status. This would be much more difficult if the vibration monitoring device or its display was integrated in the tool's casing because there is a continuous endeavour to reduce the size and the weight of hand held or hand guided tools.

According to yet another preferred embodiment it is suggested that the current vibration value is determined online during operation of the tool by means of vibration sensing means. The vibration sensing means could comprise, for example, an accelerometer. They can be located directly in the tool or on a part of the operator, for example attached to his hand or his arm. An electric signal indicative of the determined vibration value can be transmitted to the monitoring device via a cable or by means of a wireless data transmission, for example by means of a radio frequency (RF) data transmission. This has the advantage that the vibration value used for calculating the overall vibration level the operator is exposed to can be determined with a particularly high accuracy. Consequently, also the overall vibration level the operator has been exposed to and the remaining time the operator may still use the tool before reaching or exceeding the predefined permissible vibration values can be calculated very accurately. At the same time, a relatively large display unit located in the casing of the vacuum cleaner can provide the operator clearly and unambiguously with the desired information regarding the current vibration status. Alternatively, it is suggested that the vibration value is a pre-set characteristic value assigned to the tool and provided to the monitoring device prior to the calculation of the overall vibration level the operator is exposed to. The pre-set vibration value is usually determined by the manufacturer of the tool and disclosed in the tool's user manual or data sheet. It is usually determined by the manufacturer according to European Standard EN 60745. Preferably, the monitoring device comprises means for manually setting the vibration value to a value assigned to the tool, preferably to the pre-set value determined by the manufacturer of the tool prior to its use. The means for manually setting the vibration value can comprise a potentiometer or one or more push buttons, just to name two of many more possibilities.

Alternatively, it is suggested that the vibration value assigned to the tool is stored within the tool and that the means for setting the vibration value comprise means for receiving the stored vibration value assigned to the tool. For example, during or after manufacturing the tool the vibration value assigned to the tool is stored in a memory of the tool, in particular within a read-only-memory (ROM). When the overall vibration level is calculated by the monitoring device the pre-defined vibration value stored in the memory is extracted from the memory and used for the calculation of the overall vibration level. If the monitoring device makes part of a vacuum cleaner, the vibration value stored in the tool's memory has to be transmitted to the vacuum cleaner's monitoring device. This data transmission could be performed, for example, via a cable interconnecting the tool with the vacuum cleaner. It is possible to use an existing cable interconnecting the tool and the vacuum cleaner, such as an electrical power supply cable or a data transmission cable, for the transmission of the vibration value assigned to the tool and stored in the tool's memory to the monitoring device located in the vacuum cleaner. In particular, if the tool is connected to the vacuum cleaner by means of a power supply line, the vibration value assigned to the tool could be transmitted from the tool to the monitoring device by means of a power line communication (PLC).

Alternatively, it is suggested that the receiving means making part of the means for setting the vibration value in the monitoring device are adapted to receive the vibration value assigned to the tool and stored in the tool's memory via a wireless connection, in particular via a radio frequency (RF) connection. Hence, before the vibration monitoring device begins with the calculation of the overall vibration level for a working session, the stored vibration value assigned to the tool is transmitted across the wireless connection and received by the monitoring device's receiving means.

Finally, according to another preferred embodiment of the present invention, it is suggested that the vacuum cleaner is a mobile device, electrically or pneumatically driven.

Further, a hand held or hand guided tool comprising a particularly small, light-weight and simple to use monitoring device for calculating the overall vibration level the operator of the tool is exposed to during use of the tool is disclosed. This can be realized by a tool arrangement comprising a hand held or hand guided tool generating vibrations during its operation and transmitting these to an operator using the tool, the tool being activated by supplying power to the tool and deactivated by interrupting the power supply, the tool arrangement further comprising a monitoring device for calculating the overall vibration level the operator is exposed to during use of the tool, the calculation depending on a vibration value and on an exposure time value derived from a timer. The monitoring device comprises means for automatically starting the timer when power is supplied to the tool and for automatically stopping the timer after interrupting the power supply.

The hand held or hand guided tool generating the vibrations can be electrically or pneumatically driven. If the tool is electrically driven, an electric power is supplied to an electric motor of the tool via an electrical power supply line. If the tool is pneumatically driven, an air pathway for compressed air is attached to the tool so the compressed air can be applied to a pneumatic motor of the tool. The hand held or hand guided tool can be any one of: a grinder, a polisher, a sander, a planer, an edge trimmer, a saw, a glazing machine, a scouring machine, a mixer, a drill or the like all electrically or pneumatically powered and all performing a linear, a rotary, a random orbital or an orbital movement.

The idea is to omit the vibration sensor in the vibration monitoring device and nonetheless to still provide an accurate and reliable information to the operator on the current vibration status, that is regarding the current cumulative vibration level in view of the predefined maximum permissible vibration values. However, omission of the vibration sensor makes it necessary to find an alternative signal for precisely starting and/or stopping the timer. Therefore, information used for starting and/or stopping the timer is directly deduced from the tool's power supply. For example, an electrical signal indicative of whether power is being supplied to the tool or not is used for starting and stopping the timer. In particular, if power is applied to the tool, a corresponding electrical signal is generated, which starts the timer. Further, if the power supply is interrupted, the value of the previously generated electrical signal is changed or another electrical signal is generated, eventually leading to a stop of the timer. As previously mentioned, the power provided to the electric tool and used for deriving the electrical signal for automatically starting and/or stopping the timer can be an electric power or a pneumatic power. An electrical signal indicative of whether power is being supplied to the tool or not and used for automatically starting and stopping the timer could be generated by means of a magnetic flow switch which is described, for example, in EP 1 675 146 A1.

Although the starting and/or stopping of the timer is independent from the value of the actual vibrations currently generated by the tool or to which the operator is currently exposed, the vibration monitoring device still offers highly accurate information on the current vibration status because the timer measures only those periods of time during which the tool is actually turned on and actively used by the operator. This provides for an easily realizable and highly accurate device for monitoring the overall cumulative vibration level the user of a vibrating hand guided tool is exposed to, as provided, for example, in EN ISO 5349-1 and EN ISO 5349-2.

The tool can be electrically or pneumatically powered. The means for starting and/or stopping the timer comprises a power supply sensing unit in connection with a power supply conduit for providing electricity or an air-flow to the tool. The sensing unit generates an electrical signal indicative of an activation and/or a deactivation of the tool and provides the electrical signal to the timer for starting and/or stopping the timer. The electrical signal is directly derived from whether or not power is provided to the tool. For example, if the tool is pneumatically driven, it could be possible to locate a flow-rate meter in the air pathway feeding the compressed air to the pneumatic tool. The flow-rate meter measures the flow rate of the compressed air passing through the pathway and generates a corresponding electric signal, which can be used for starting and/or stopping the timer. In the case of an electrically driven tool a current sensing unit could be located in the electrical power supply line providing the tool with electric power in order to sense the current flow and to generate a corresponding electric signal used for starting and/or stopping the timer. Of course, the air flow measuring device and/or the current sensing unit could be located at any point along the power supply conduit, even within the tool.

Furthermore, the electric signal for starting and/or stopping the timer could also be generated depending on the switching position of a power switch for activating and deactivating the tool. If the tool is activated, the power switch is brought into an ON-position, and when the tool is deactivated, the power switch or brought into an OFF-position. Therefore, the switching position of the power switch is directly indicative of the activation and/or deactivation of the tool and, therefore, can be used for starting and/or stopping the timer according to the present invention.

It is suggested that the tool is connected to a vacuum cleaner for aspiring dust generated during operation of the tool, the vacuum cleaner being automatically activated upon activation of the tool and automatically deactivated after deactivation of the tool depending on an electric signal, the electrical signal also being provided to the timer for automatically starting and/or stopping the timer. The electric signal is not only used to activate and/or deactivate the vacuum cleaner depending on the current operating status of the tool (activated or deactivated) but also for starting and/or stopping the timer. Hence, an electrical signal which is present in the tool arrangement anyway can be used for starting and/or stopping the timer. No additional measuring or sensing units located in the power supply conduit have to be provided in order to generate the electrical signal for starting and/or stopping the timer.

The timer can be an integral part of the vibration monitoring device. Further, the vibration monitoring device and the timer could be an integral part of the hand held or hand guided tool generating the vibrations. Alternatively, the vibration monitoring device and the timer could be located separately from the tool. For example, the vibration monitoring device and the timer could be attached to or integrated in the vacuum cleaner. In that case the vibration monitoring device and the timer are preferably an integral part of the vacuum cleaner, for example located in a casing of the vacuum cleaner. Preferably, the integration of the monitoring device is such that the current vibration value, the cumulated vibration level and/or the remaining time of use before a tolerable maximum cumulated vibration level has been passed is easily visible for the operator of the hand held or hand guided tool.

The vibration monitoring device preferably has at least one display for providing information regarding the vibrations to the operator of the tool. For example, the display could output to the operator information regarding the cumulative time of use of the tool or a remaining time for which the operator can go on using the tool until the pre-defined permissible cumulative maximum vibration levels are reached. The display could be realized as any kind of analogue or digital display. The information output to the operator by the display could be toggled, thereby switching the display between different types of information presented to the operator. For example, the display could be a LCD- or LED-display. The display could be adapted to output the information by means of numerals only or graphically. Of course, other types of displays can be used for the vibration monitoring device, too, without departing from the present invention.

The vibration value characteristic of the tool generating the vibrations can be a predefined or pre-set value assigned to the tool and provided to the monitoring device prior to the calculation of the overall vibration level. The pre-set vibration value characteristic for the tool could be stored in a memory unit making part of the monitoring device prior to the intended use of the tool. Preferably, the vibration monitoring device comprises means for manually setting or inserting the characteristic vibration value assigned to the tool. The characteristic vibration value assigned to the tool can be determined beforehand by the manufacturer of the tool by means of theoretical calculation of the vibrations and/or by measurement of the actual vibrations practically occurring during use of the tool.

Further features and advantages of the present invention will become apparent from the following description of the figures. Of course, the present invention has the described features and advantages not only in the described combination. Rather, the various features of the described embodiments can be freely combined with one another without departing from the present invention. The figures show:
- Figure 1: a tool arrangement in a schematic view;
- Figure 2: a top view of an example for a tool making part of the tool arrangement of figure 1;
- Figure 3: a perspective view of an example for a tool making part of the tool arrangement of figure 1;
- Figures 4a to 4d: various views of a separate vibration monitoring device not making part of the invention;
- Figures 5a to 5d: various views of another separate vibration monitoring device not making part of the invention;
- Figures 6a to 6d: various views of yet another separate vibration monitoring device not making part of the invention; and
- Figure 7: a preferred embodiment of a vacuum cleaner according to the present invention.

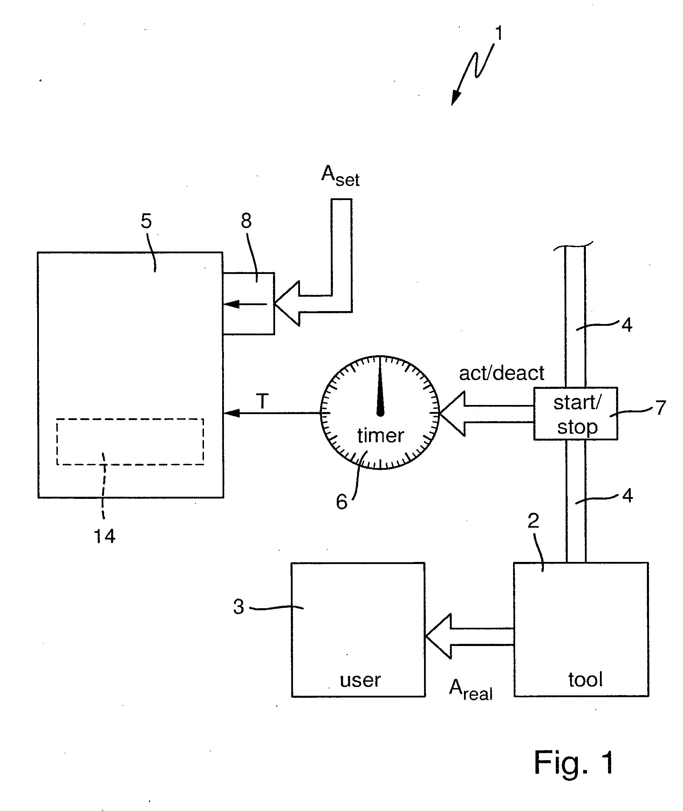

In figure 1 a tool arrangement is shown in a schematic view. The tool arrangement of figure 1 is generally designated with reference sign 1. The preferred embodiment of the tool arrangement 1 of figure 1 comprises a hand held or hand guided tool 2 generating vibrations Aᵣₑₐₗ during its operation. The vibrations Aᵣₑₐₗ are transmitted to an operator 3 using the tool 2. The tool 2 is activated by supplying power to the tool 2 and deactivated by interrupting the power supply. An ON/OFF-switch (not shown) can be provided at the tool 2 for activating and deactivating the tool. The power for activating the tool 2 can be an electrical power provided to the tool 2 by means of a power supply line connecting the electrical tool 2 with an electrical power supply source. Alternatively, the power for activating the tool 2 could be compressed air provided to the tool 2 by means of an air pathway connecting the pneumatic tool 2 with a pneumatic power supply source, for example an accumulator for compressed air provided thereto by a compressor or the like (not shown). In figure 1 a conduit between the tool 2 and a power supply source is generally designated with reference sign 4. The conduit 4 can be an electrical power supply line or a pneumatic air pathway for compressed air.

The tool arrangement 1 further comprises a monitoring device 5 for calculating the overall vibration level the operator 3 is exposed to during use of the tool 2. The calculation of the overall vibration level depends on a vibration value A and on an exposure time value T derived from a timer 6.

Tool arrangements of this type are well known in the prior art. It is known to measure the vibration value Aᵣₑₐₗ used for calculating the overall vibration level by means of a vibration sensing device located in or on the tool 2 or on a part of the user 3, for example on his hand or arm. It is further known to start and stop the timer 6 from which the exposure time value T is derived depending on the current vibration value measured by the vibration sensing device. Hence, in the prior art the timer 6 is started if the measured vibration value reaches or exceeds a pre-determined threshold value and is stopped if the measured vibration value reaches or goes below a pre-determined threshold value. Hence, in the prior art it is attempted to determine the overall vibration level as accurately as possible by exactly measuring the actual vibration value Aᵣₑₐₗ by means of the vibration sensing device. The vibration sensing device is also used for starting and stopping the timer for determining the exposure time value.

The provision of such a vibration sensing device and the processing of the signals generated by such a vibration sensing device makes the tool arrangement 1 in particular the monitoring device 5 rather complex, large and heavy in design and complicated in use. Of course, the known monitoring devices 5 and timers 6 are capable of providing a rather exact value for the overall vibration level the operator 3 is exposed to during use of the tool 2. However, during practical use of the tool 2 by an operator 3 there are much more factors having an impact on the overall vibration level. Hence, the prior art offers a merely theoretical possibility for enhancing the accuracy of the calculated value for the overall vibration level. Practically, no significant enhancement of the accuracy of the calculated overall vibration level the operator 3 is exposed to during use of the tool 2 can be achieved because of a plurality of other factors influencing the calculated overall vibration level and not being regarded in the prior art. Additionally, in the prior art the vibration monitoring devices have to be carried by the user, either as part of the tool 2 or directly on his hand or arm.

What is clearly needed is a tool arrangement 1 comprising a vibration monitoring device 5, which has a small size, a light weight, which is easy to use and which does not attempt to offer a theoretically highly accurate value for the overall vibration level but rather gives a good and reliable estimation of the overall vibration level and of the remaining time the operator 3 can continue to use the tool 2 under European Standard EN 60745 before running the risk of exceeding the allowed vibration threshold values defined therein.

Therefore, the monitoring device 5 comprises means 7 for automatically starting the timer 6 when power is supplied to the tool 2 and/or for automatically stopping the timer 6 when the power supply is interrupted. Preferably, the means 7 for starting/stopping the timer 6 generate an electrical signal act/deact for starting/stopping the timer 6. The electrical signal act/deact is directly derived from a measure indicative of the activation and/or deactivation of the tool 2. Preferably, the electrical signal act/deact is directly derived from the activation/deactivation of the power supply to the tool 2. The electrical signal act/deact is independent from the actual value Aᵣₑₐₗ of the vibrations currently generated by the tool 2 or to which the operator 3 is currently exposed.

Preferably, the means 7 for starting/stopping the timer 6 are located at least partially within the conduit 4 and comprise a power supply sensing device, which is adapted to sense when power (electricity or compressed air) is supplied to the tool 2 and when the power supply is interrupted. Depending on the sensed status of the power supply (active: power supply to the tool 2/ inactive: power supply is interrupted) the electrical signal act/deact is generated by the means 7 and applied to the timer 6 for starting and/or stopping the timer 6. The means 7 may comprise a current sensor or an air-flow sensor. Alternatively, the means 7 could comprise an ON/OFF-switch for supplying power/ interrupting power supply to the tool 2. In that case the electrical signal for starting/stopping the timer 6 is generated according to the switching position of the ON/OFF-switch.

In the monitoring device 5 no vibration sensing device for measuring the actual value Aᵣₑₐₗ of the vibrations currently generated by the tool 2 and no calculating means for comparing the measured actual value Aᵣₑₐₗ with pre-defined threshold values are necessary for determining start and stop conditions for the timer 6. Rather, the start/stop conditions of the timer 6 are directly derived from a measure indicative of the activation/deactivation of the tool 2. Therefore, the tool arrangement 1 has no need for a vibration sensing device. This makes the tool arrangement 1 and in particular the monitoring device 5 less complex, easier to use, smaller and lighter.

Further, the practical usability of the tool arrangement 1 is very good without any constraints regarding the informative value of the calculated overall vibration level for the operator 3. The operator 3 is provided with a good and reliable estimation of the overall vibration level which allows him to easily determine whether and for how long he may continue to use the tool 2 generating the vibrations before the pre-defined vibration threshold values according to EN 60745 are reached or even exceeded. A few minutes more or less of further use of the tool 2 are not essential in this respect. Although other tool arrangements and monitoring devices may provide a more accurate value of the overall vibration levels and of the possible remaining time of use, the tool arrangement 1 and the monitoring device 5 described above provide a sufficiently accurate overall vibration level to the user 3 of the tool 2.

Due to the omission of a vibration sensing device in the tool arrangement 1, the vibration value is not measured online during the use of the tool 2. Rather, a pre-defined vibration value Aₛₑₜ assigned to the tool 2 is provided to the monitoring device 5 prior to the calculation of the overall vibration level. Preferably, the monitoring device 5 comprises means 8 for setting the vibration value to a value Aₛₑₜ characteristic of the tool 2. The means 8 for setting the vibration value may comprise means for manually setting the characteristic vibration value, in particular a potentiometer or one or more push-buttons. By means of these, the pre-set vibration value Aₛₑₜ can be entered into the monitoring device 5 so the pre-set vibration value Aₛₑₜ can be taken into consideration during the calculation of the overall vibration level. The pre-set vibration value Aₛₑₜ can be determined (calculated or experimentally measured) beforehand by a manufacturer of the tool 2 and is usually mentioned in the tool's 2 user manual and/or data sheet. Preferably, the pre-set vibration value Aₛₑₜ is determined in accordance with European Standard EN 60745.

Alternatively, the pre-set vibration value Aₛₑₜ is stored in a memory unit of the tool 2. The memory could be, for example, some kind of read-only-memory (ROM). Before calculation of the overall vibration level, for example at the beginning of a working session, the vibration value Aₛₑₜ is transmitted to the monitoring device 5. In that case the means 8 for setting the vibration value may comprise means for receiving the characteristic vibration value from the tool 2 or its memory, respectively. The transmission can be effected by means of a data-transmission cable running between the tool 2 and the monitoring device 5, or by means of a wireless data link, for example a radio frequency (RF) link, established between the tool 2 and the monitoring device 5.

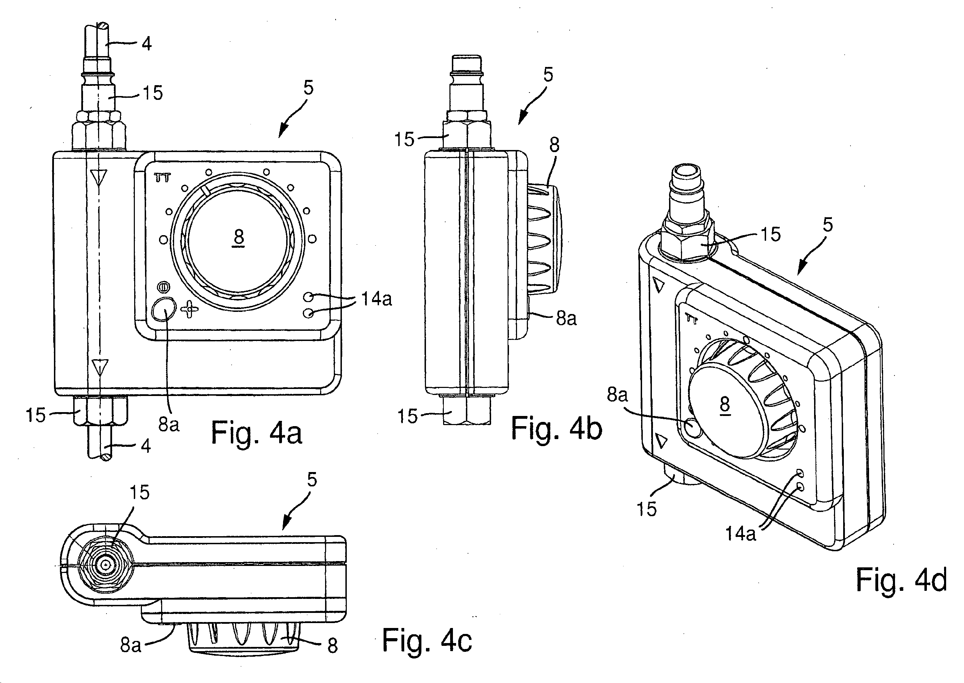

The monitoring device 5, the timer 6 and the means 7 for starting/stopping the timer 6 can be separate units located at different positions within the tool arrangement 1, or integrated in a single unit (for example see the embodiments of figures 4 to 6). The single unit comprising the monitoring device 5, the timer 6 and/or the means 7 for starting/stopping the timer 6 can be located separate from the tool 2 and/or of other components (for example a vacuum cleaner) connected to the tool 2. In particular, any, some or all of the units 5, 6, 7 can form an integral part of the tool 2 or of a component connected to the tool 2, such as a vacuum cleaner.

The hand held or hand guided tool 2 making part of the tool arrangement 1 may be any tool generating vibrations during its use, in particular one of: a grinder, a polisher, a sander, a planer, an edge trimmer, a saw, a glazing machine, a scouring machine, a mixer, or a drill. As mentioned before, the tool 2 can be electrically or pneumatically powered. Furthermore, the tool 2 can perform a linear, a rotary, a random orbital or an orbital movement. The movement of the tool's 2 working element provokes the vibrations, which are transmitted to the operator 3.

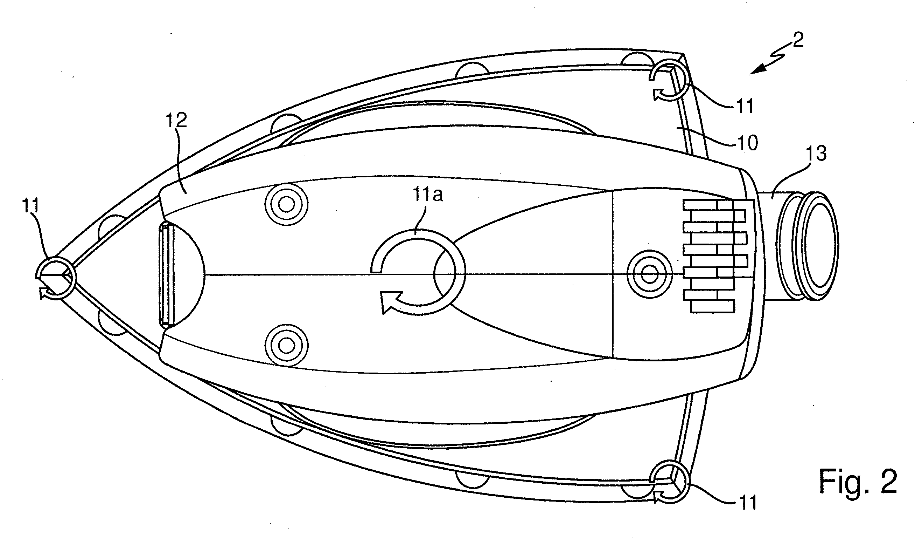

Figure 2 shows a top view of an exemplary embodiment of a tool 2, which can be used in connection with the tool arrangement 1. The tool 2 of figure 2 is designed as an orbital sander but, of course, can be designed as any hand-held or hand-guided machine tool 2 generating and implying onto an operator 3 vibrations. The orbital sander 2 of figure 2 comprises a working element 10 adapted to perform an orbital movement indicated by circular arrows 11. An orbital movement 11 of the working element 10 means that it moves in two directions extending perpendicular to one another and defining the planar extension of the working element 10. The movement 11 of the working element 10 provokes vibrations, generally designated by circular arrow 11a. The tool 2 comprises a housing 12, preferably made of an artificial material, in particular of a plastic material. The housing 12 is formed such that it can be conventionally grasped by the operator 3 with one or two hands. Hence, the housing 12 also serves as a grip or handle of the tool 2 for safely guiding the tool 2 along a working surface of a work piece. By holding the tool 2 at its housing 12 at least part of the vibrations 11a are transmitted to the operator 3.

The working element 10 is movably connected to the hosing 12 in order to allow an orbital movement 11 of the working element 10 in respect to the machine tool's housing 12. In its inside the housing 12 comprises a motor (not shown) for actuating the machine tool 2, in particular for provoking the working element's 10 movement 11. The motor can be an electrically or a pneumatically driven motor. In the present embodiment the motor is an electric motor. The tool 2 is designed such that dust, which is generated during use of the tool 2, is absorbed through suction holes and suction channels (both not shown) provided within the working element 10 and conveyed to a discharge vent 13. A vacuum cleaner (not shown) can be connected to the vent 13 by means of an extraction hose (not shown) for dust collection or filtering.

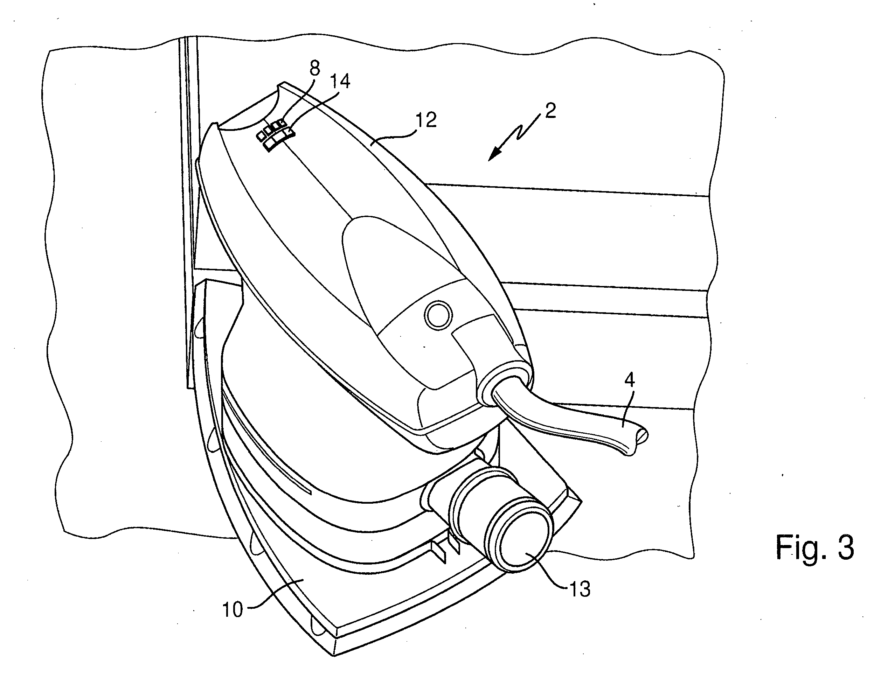

Depending on whether the tool 2 is electrically or pneumatically driven, the conduit 4 (see figure 3) for power supply is an electrical power line or an air pathway for compressed air. In the present case the conduit 4 is an electric power supply line. One or more of the units, that is the monitoring device 5, the timer 6 and/or the means for starting/stopping the timer 6, can be integrally formed as part of the tool 2. For example, on top of the casing 12 the embodiment of figure 3 shows the means 8 for manually setting the pre-set vibration value Aₛₑₜ assigned to the tool 2. The means 8 are embodied as four push-buttons. Of course, the means 8 for manually setting the pre-set vibration value Aₛₑₜ can comprise more or less than the shown four push-buttons. Additionally or alternatively, the means 8 can comprise different input-means other than push-buttons, for example one or more potentiometers. Furthermore, on top of the casing 12 of the tool 2 shown in figure 3 output-means in the form of a display 14 are shown. The display 14 serves for outputting information to the operator 3 using the tool. The information refers to the vibration status, that is in particular to the overall vibration level the operator 3 has been exposed to up to now during the current working session, the time the operator 3 has been exposed to the vibrations and/or the time still remaining during which the operator 3 would be able to further use the tool 2 without running the risk of reaching or exceeding the pre-defined allowable threshold values. Of course, any other vibration-related information could be output on the display 14, too.

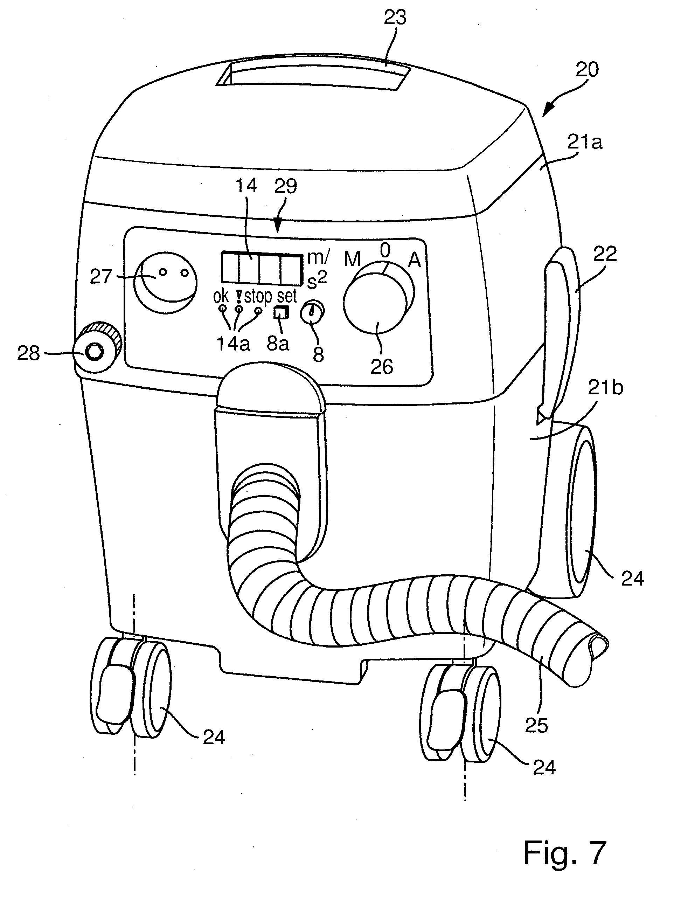

In the embodiment of figure 1, the display 14 is shown only with dashed lines in order to make it clear that the display 14 is merely facultative to the monitoring device 5. Instead or additionally to a display 14 it is also possible to provide some kind of audio and/or video indicators for providing the operator 3 with the desired information regarding the overall vibration level. For example, the monitoring device 5 could be provided with three indicator lights 14a (see figure 7), one of them green for indicating an overall vibration level well below the pre-defined allowed threshold value ("OK"), one of the indicator lights being yellow indicating a cumulative vibration level near to the allowed pre-defined threshold value ("!"), and one of the indicator lights being red for indicating an overall vibration level which has already exceeded the allowed pre-defined threshold value ("STOP").

The monitoring device 5 comprises means for processing data, for example a microprocessor or a microcontroller, and a computer program running on the processing means (not shown). During execution on the processing means the computer program realizes the calculation of the overall vibration level the operator 3 has been exposed to so far based on the pre-defined vibration value Aₛₑₜ and the current value of the exposure time T. The processing means and other elements making part of the monitoring device 5 are preferably located within the casing 12 of the tool 2 and not visible from outside. The timer 6 preferably makes part of the monitoring device 5. The means 7 for starting/stopping the timer 6 are preferably located at some point within the casing 12 of the tool 2 where the power supply via the conduit 4 can be easily monitored. The electrical signal act/deact for starting and/or stopping the timer 6 is forwarded from the means 7 to the timer 6 entirely within the casing 12 of the tool 2.

As an alternative to the embodiment of figure 3, the monitoring device 5, the timer 6 and the means 7 for starting and/or stopping the timer 6 are preferably located separately from the tool 2. Such an embodiment of the tool 2 is shown in figure 2. In that case a monitoring device 5 is used, which is separate from the tool 2, like the different embodiments of a monitoring device 5 shown in figures 4 to 6. The timer 6 and the means 7 for starting/stopping the timer 6 are preferably integrally formed with the monitoring device 5.

Figure 4a shows a front view of a monitoring device 5 designed separately from the tool 2. Figure 4b shows a side view, figure 4c a top view and figure 4d a perspective view of the monitoring device 5 of figure 4a. The monitoring device 5 can be located directly in the conduit 4 through which electric current or compressed air is provided to the tool 2. In particular, the upper part of the conduit 4 in figures 4a to 4c is connected to an energy source (an electric or pneumatic source) and the lower part of conduit 4 is connected to the tool 2. The monitoring device 5 comprises means 8 for manually setting the pre-set vibration value Aₛₑₜ assigned to the tool 2. The means 8 for manually setting the pre-set vibration value Aₛₑₜ comprise an adjusting knob connected to a potentiometer or the like. Furthermore, the monitoring device 5 is provided with a push-button 8a for confirming the pre-set vibration value Aₛₑₜ and/or for starting operation of the monitoring device 5. Further, the monitoring device 5 comprises two indicator lights 14a, preferably colored, for indicating the current status of the monitoring device 5 and/or for outputting to the operator 3 vibration-related information, like information relating to the current overall vibration level.

The monitoring device 5 comprises two attachments 15, by means of which the monitoring device 5 can be located directly within the conduit 4 for providing the tool 2 with electric or pneumatic power, that is within an electrical power line (for an electrically driven tool 2) or within an air pathway (for a pneumatically driven tool 2). The means 7 for sensing an activation or deactivation of the power for the tool 2 and for generating the electrical signal act/deact for starting/stopping the timer 6 as well as the timer 6 itself are preferably an integral part of the monitoring device 5. The means could be, for example, a magnetic flow switch of the type disclosed in EP 1 675 146 A1, which can be located within an air or current pathway through the monitoring device 5, respectively. The timer 6 provides the monitoring device 5 or its processing means, respectively, with the current value of the exposure time T. Preferably, the embodiment shown in figures 4a to 4d is designed for use in connection with a pneumatically driven tool 2. Hence, the conduit 4 is an air pathway for compressed air eventually operating the tool 2.

The pre-set vibration value Aₛₑₜ is provided to the monitoring device 5 or its processing means, respectively, from the input means 8. The processing means of the monitoring device 5 calculate the overall cumulative vibration level depending on the pre-set vibration value Aₛₑₜ and the exposure time T. This calculation is performed continuously during operation of the tool 2, preferably at discrete points in time depending on the processing means' processing cycle, for example every 5 milliseconds. Information regarding the overall vibration level can be communicated to the operator 3 by means of the indicator lights 14a and/or acoustically.

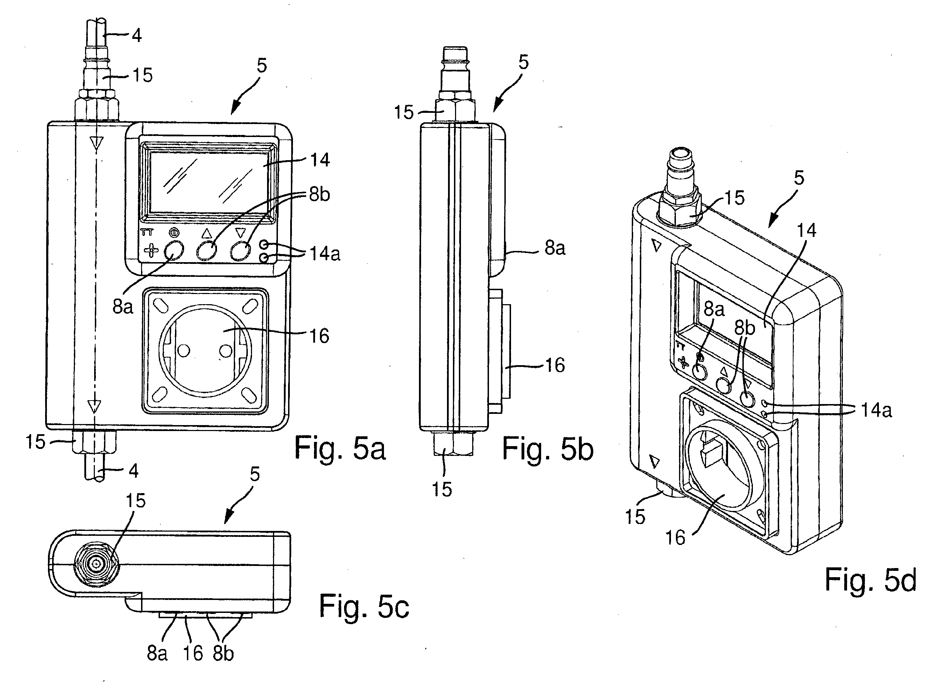

Figure 5a shows a front view of another embodiment of a monitoring device 5 designed separately from the tool 2. Figure 5b shows a side view, figure 5c a top view and figure 5d a perspective view of the monitoring device 5 of figure 5a. The monitoring device 5 according to figures 5a to 5d essentially corresponds to the preceding embodiment according to figures 4a to 4d as far as its principal functionality is concerned. One difference between these two embodiments is that the embodiment of figures 5a to 5d comprises an additional electric socket 16 into which an external electrically driven component, for example a separate vacuum cleaner (see figure 7), can be inserted. The electric socket 16 is provided with electrical power whenever the monitoring device 5 senses a power supply to the tool 2 through the conduit 4. Hence, the vacuum cleaner or any other electrical component plugged into socket 16 could be activated synchronously with the tool 2, which is activated by electrical current or compressed air running through the conduit 4.

Alternatively, an electrically driven tool 2 is plugged into the electric socket 16. The electric power is provided to the monitoring device 5 and consequently to the electric socket 16 by means of the conduit 4 (embodied as an electrical power line) or by means of a separate electrical power line (not shown in figures 5a to 5d). In the latter case the monitoring device 5 is adapted for use with a pneumatically driven tool 2 (provided with compressed air via the air pathway 4) or alternatively with an electrically driven tool 2 (provided with electric current via the separate electrical power line and the socket 16). If an electric power tool 2 is plugged into the socket 16, activating the tool 2 will cause the monitoring device 5 or appropriate means provided therein to generate the electrical signal act/deact used for automatically starting and stopping the timer 6.

Furthermore, apart from the indicator lights 14a the monitoring device 5 of figures 5a to 5d is provided with a fully functional display 14 for outputting vibration-related information to the operator 3. The outputted information may be, for example, information relating to the overall vibration level calculated by the monitoring device 5 or its processing means, respectively. Further, the means 8 for manually setting the pre-set vibration value Aₛₑₜ comprise two push buttons 8b, one for increasing the pre-set value and one for decreasing the pre-set value. The currently set vibration value can be output to the operator 3 by means of the display 14. If the currently set vibration value corresponds to the pre-set vibration value Aₛₑₜ assigned to the tool 2, that is if the pre-set value Aₛₑₜ has been correctly entered into the monitoring device 5, the input can be confirmed by pushing one or more appropriate push-buttons, for example push-button 8a, or in any other way. Then processing of the input data Aₛₑₜ and T by means of the monitoring device 5 or its processing means, respectively, and calculation of the overall vibration level could be started.

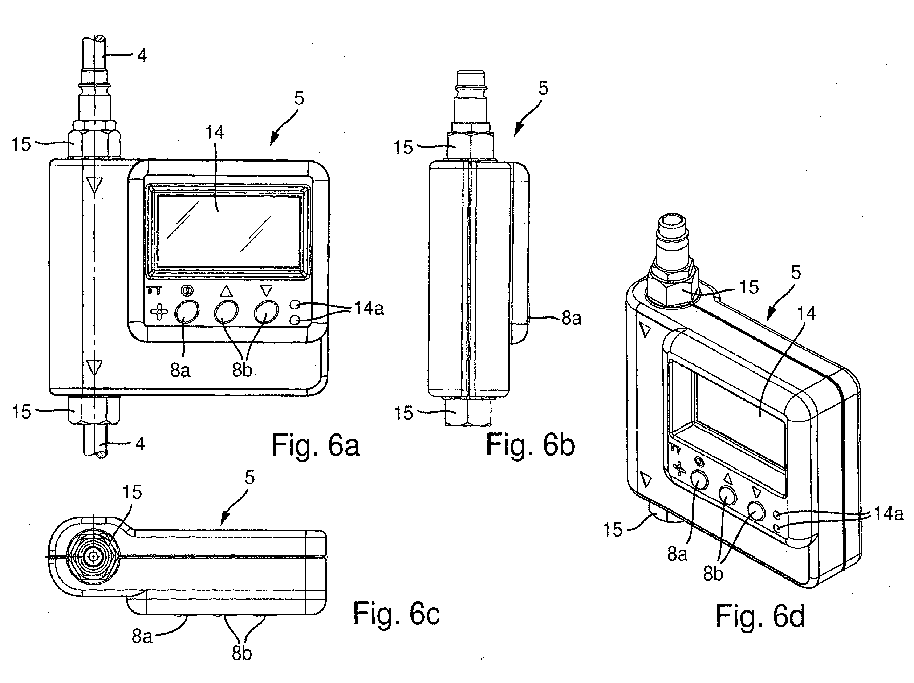

Figure 6a shows a front view of another embodiment of a monitoring device 5 designed separately from the tool 2. Figure 6b shows a side view, figure 6c a top view and figure 6d a perspective view of the monitoring device 5 of figure 6a. The embodiment shown in figure 6a to 6d essentially corresponds to the embodiment of figures 4a to 4d apart from the fact that an additional display 14 is provided for outputting vibration-related information to the operator 3. 8b. Further, the means 8 for manually setting the pre-set vibration value Aₛₑₜ comprise two push buttons 8b, one for increasing the pre-set value and one for decreasing the pre-set value. Preferably, the monitoring device 5 is adapted for use in connection with a pneumatically driven tool 2. Hence the conduit 4 is embodied as an air pathway for compressed air. Of course, like the embodiment of figures 4a to 4d, this embodiment could also be used in connection with an electrically driven tool 2. Hence the conduit 4 would be embodied as an electrical power line for electric current.

Finally, figure 7 shows a vacuum cleaner according to the present invention. The vacuum cleaner is generally designated with reference sign 20. The vacuum cleaner 20 comprises an essentially two-part casing comprising an upper part 21a and a lower part 21b. The lower part 21b of the casing comprises a collecting tank or dust bin for storing what has been aspired by the vacuum cleaner 20. The top part 21a of the casing comprises among others a motor for creating an underpressure (i.e. a pressure below the ambient pressure) and for making the vacuum cleaner 20 aspire dust and the like. Furthermore, the top part 21a of the casing may comprise one or more air filters and control and display elements 26 for the operational control of the vacuum cleaner 20. The control elements 26 in particular comprise a rotary control switch adapted for turning on and off the vacuum cleaner 20, for switching between automatic and manual operational mode and/or for speed control. In the automatic mode a power plug of the electrical power supply line 4 of an electrical tool 2 is inserted into power socket 27 of the vacuum cleaner 20. The vacuum cleaner 20 for his part is connected to an electrical power supply used for providing the vacuum cleaner 20 as well as the tool 2 with electrical power. If the tool 2 is turned on the vacuum cleaner 20 is automatically turned on, too, whereby the electrical power for the tool 2 is provided by the vacuum cleaner 20. Alternatively, in the automatic mode an air plug of the air pathway 4 of a pneumatic tool 2 is inserted into an air socket 28 of the vacuum cleaner 20. The vacuum cleaner 20 for his part is connected to an electrical power supply used for providing the vacuum cleaner 20 with electrical power. Additionally, the vacuum cleaner 20 is connected to a compressed air supply for providing the tool 2 with compressed air. If the tool 2 is turned on compressed air is provided to the tool 2 via the air socket 28, and the vacuum cleaner 20 is automatically turned on synchronously to the activation of the tool 2. Furthermore, it is possible to drive the vacuum cleaner 20 or its motor, respectively, with compressed air, too, instead of electrical power. In that case the tool 2 as well as the vacuum cleaner 20 would be driven by compressed air.

The top part 21a of the casing is releasably connected to the bottom part 21b by means of vice action latches 22 located at two opposite sides of the casing 21a, 21b. The top part 21a of the casing is provided with a handle 23 for conveniently carrying the vacuum cleaner 20 to its designated site of operation. The bottom part 21b of the casing is provided with wheels 24 so the vacuum cleaner 20 can be conveniently rolled from one position to another. Preferably, the front wheels are pivotable about an essentially vertical pivoting axis in order to allow easy maneuvering of the vacuum cleaner 20 and comprise braking means for securing the vacuum cleaner 20 in the desired site of operation.

The vacuum cleaner 20 is adapted for connection to a hand held or hand guided tool, such as tool 2 shown in figures 2 and 3, generating vibrations during its operation and transmitting these to an operator 3 using the tool 2. The vacuum cleaner 20 is connected to the tool 2 by means of an aspiration hose 25 one end of which is inserted into the bottom part 21b of the casing of the vacuum cleaner 20. The opposite end of the hose 25 is connected to the dust vent 13 (see figures 2 and 3) of the tool 2. The vacuum cleaner 20 serves for aspiring dust generated during operation of the tool 2. Preferably, the vacuum cleaner 20 is automatically activated upon activation of the tool 2 and automatically deactivated after deactivation of the tool 2 by means of an electrical signal. This electrical signal can be directly derived from whether or not electrical power is transmitted to the tool 2 via the power supply line connecting the tool 2 and the vacuum cleaner 20 via the power socket 27. The means 7 for detecting whether or not electrical power is transmitted to the tool 2 are preferably located in the vacuum cleaner 20, too.

Finally, the vacuum cleaner 20 also comprises a display and control panel 29 of the monitoring device 5 well visible to an operator 3. The panel 29 comprises three indicator lights 14a, one of them green for indicating an overall vibration level well below the pre-defined allowed threshold value ("OK"), one of the indicator lights being yellow indicating a vibration level near to the allowed pre-defined threshold values ("!"), and one of the indicator lights being red for indicating an overall vibration level which has already exceeded the allowed pre-defined threshold values ("STOP"). Further, the panel 29 comprises means 8 for manually setting the pre-set vibration value Aₛₑₜ in the form of an adjusting knob connected to a potentiometer or the like. Furthermore, the panel 29 of the monitoring device 5 is provided with a push-button 8a for confirming the pre-set vibration value Aₛₑₜ and/or for starting operation of the monitoring device 5 or its processing means, respectively. Finally, the panel 29 also comprises a display 14 for outputting to the operator 3 vibration-related information.

The vacuum cleaner 20 according to the present invention has the advantage that the monitoring means 5, the means 7 for sensing power-on and/or power-off of the tool 2 and for generating the electrical signal act/deact for starting and/or stopping the timer as well as the timer 6 can be easily incorporated. If the vacuum cleaner 20 is connected to an electrical power supply, no additional electrical power supply has to be provided for the monitoring device 5, the timer 6 and the means 7. Additionally, the casing 21a, 21b of the vacuum cleaner 20 offers enough free surface for providing the display and control panel 29 thereon. The means 8, 8a for setting the pre-set vibration value Aₛₑₜ assigned to the tool 2. can be located on the panel 29 and designed such that they can be easily actuated by the operator 3. Further, the display 14 and the indicator lights 14a can be located and designed such that they are clearly and easily visible to the operator 3. Finally, the monitoring device 5 used in the tool arrangement 1 and/or the vacuum cleaner 20 according to the present invention does not make use of a vibration sensor. Rather, the timer 6 for providing the exposure time value T is started and/or stopped by an electrical signal act/deact which is directly derived from whether or not power is supplied to the tool 2 independently for the actual current vibration values generated by the tool 2 or to which the operator 3 is exposed to.

## Claims

1. Vacuum cleaner (20) adapted for connection to a hand held or hand guided tool (2) generating vibrations during its operation and transmitting these to an operator (3) using the tool (2) and for aspiring dust generated during operation of the tool (2), wherein the vacuum cleaner (20) is automatically activated upon activation of the tool (2) and automatically deactivated after deactivation of the tool (2) by means of an electrical signal,
**characterized in that** the vacuum cleaner (20) comprises a monitoring device (5) for calculating the overall vibration level the operator (3) is exposed to during use of the tool (2), the calculation depending on a vibration value (A_set) indicative of the quantity of vibrations generated by the tool (2) during its operation and on an exposure time value (T) indicative of the duration the operator (3) is exposed to the vibrations.

2. Vacuum cleaner (20) according to claim 1, wherein the exposure time value is derived from a timer (6), which is automatically started and stopped according to the electrical signal.

3. Vacuum cleaner (20) according to claim 2, wherein the monitoring device (5) and the timer (6) are integrally formed with a casing (21a, 21b) of the vacuum cleaner (20).

4. Vacuum cleaner (20) according to one of claims 1 to 3, wherein the vibration value is determined online during operation of the tool (2) by means of vibration sensing means.

5. Vacuum cleaner (20) according to one of claims 1 to 3, wherein the vibration value is a pre-set value (A_set) assigned to the tool (2) and provided to the monitoring device (5) prior to the calculation of the overall vibration level.

6. Vacuum cleaner (20) according to claim 5, wherein the monitoring device (5) comprises means (8) for setting the vibration value to a value (A_set) assigned to the tool (2).

7. Vacuum cleaner (20) according to claim 6, wherein the means (8) for setting the vibration value comprise means for manually setting, in particular a potentiometer (8) or one or more push-buttons (8a, 8b).

8. Vacuum cleaner (20) according to claim 6, wherein the vibration value assigned to the tool (2) is stored within the tool (2) and the means (8) for setting the vibration value comprise means for receiving the stored vibration value assigned to the tool (2).

9. Vacuum cleaner (20) according to claim 8, wherein the receiving means are adapted to receive the vibration value assigned to the tool (2) across a wireless connection, in particular across a radio-frequency connection.

10. Vacuum cleaner (20) according to one of claims 1 to 9, wherein the vacuum cleaner (20) is a mobile device, electrically or pneumatically driven.

## Patentansprüche

1. Staubsauger (20), angepasst zum Anschließen an ein in der Hand gehaltenes oder von Hand geführtes Werkzeug (2), das während seines Betriebes Schwingungen erzeugt und diese zu einem das Werkzeug (2) benutzenden Bediener (3) überträgt, und zum Ansaugen von Staub, der während des Betriebes des Werkzeugs (2) anflällt, wobei der Staubsauger (20) mittels eines elektrischen Signals bei Aktivierung des Werkzeugs (2) automatisch aktiviert wird und nach Deaktivierung des Werkzeugs (2) automatisch deaktiviert wird, **dadurch gekennzeichnet, dass** der Staubsauger (20) eine Überwachungsvorrichtung (5) zum Berechnen des Gesamtschwingungspegels, dem der Bediener (3) während der Benutzung des Werkzeugs (2) ausgesetzt ist, aufweist, wobei die Berechnung von einem Schwingungswert (A_set), der auf die Anzahl der vom Werkzeug (2) während seines Betriebes erzeugten Schwingungen hinweist, und von einem Wert (T) für die Dauer der Aussetzung, der auf die Dauer hinweist, für die der Bediener (3) den Schwingungen ausgesetzt ist, abhängt.

2. Staubsauger (20) nach Anspruch 1, wobei der Wert für die Dauer der Aussetzung von einem Zeitglied (6), das entsprechend dem elektrischen Signal automatisch gestartet und gestoppt wird, abgeleitet wird.

3. Staubsauger (20) nach Anspruch 2, wobei die Überwachungsvorrichtung (5) und das Zeitglied (6) vollständig mit einem Gehäuse (2 1 a, 21b) des Staubsaugers (20) gebildet werden.

4. Staubsauger (20) nach einem der Ansprüche 1 bis 3, wobei der Schwingungswert online während des Betriebes des Werkzeugs (2) mittels Schwingungsabtasteinrichtungen bestimmt wird.

5. Staubsauger (20) nach einem der Ansprüche 1 bis 3, wobei der Schwingungswert ein vorgegebener, dem Werkzeug (2) zugewiesener Wert (A_set) ist und der Überwachungsvorrichtung (5) vor der Berechnung des Gesamtschwingungspegels zur Verfügung gestellt wird.

6. Staubsauger (20) nach Anspruch 5, wobei die Überwachungsvorrichtung (5) Mittel (8) zum Einstellen des Schwingungswertes auf einen dem Werkzeug (2) zugewiesenen Wert (A_set) aufweist.

7. Staubsauger (20) nach Anspruch 6, wobei die Mittel (8) zum Einstellen des Schwingungswertes Mittel zum manuellen Einstellen, insbesondere ein Potentiometer (8) oder einen oder mehr Drucktaster (8a, 8b), aufweisen.

8. Staubsauger (20) nach Anspruch 6, wobei der dem Werkzeug (2) zugewiesene Schwingungswert innerhalb des Werkzeugs (2) gespeichert wird und die Mittel (8) zum Einstellen des Schwingungswertes Mittel zum Empfangen des gespeicherten, dem Werkzeug (2) zugewiesenen Schwingungswertes aufweisen.

9. Staubsauger (20) nach Anspruch 8, wobei die empfangenden Mittel angepasst sind, den dem Werkzeug (2) zugewiesenen Schwingungswert über eine drahtlose Verbindung, insbesondere über eine Hochfrequenzverbindung, zu empfangen.

10. Staubsauger (20) nach einem der Ansprüche 1 bis 9, wobei der Staubsauger (20) ein mobiles Gerät mit elektrischem oder pneumatischem Antrieb ist.

## Revendications

1. Aspirateur (20) destiné à être relié à un outil maintenu ou guidé manuellement (2) générant des vibrations pendant son fonctionnement et les transmettant à un opérateur (3) utilisant l'outil (2) et à aspirer la poussière générée au cours du fonctionnement de l'outil (2), cet aspirateur (20) étant automatiquement activé suite à l'activation de l'outil (2) et automatiquement désactivé après la désactivation de l'outil (2) au moyen d'un signal électrique, **caractérisé en ce que**
l'aspirateur (20) comprend un dispositif de contrôle (5) permettant de calculer le niveau de vibration global auquel l'opérateur (3) est exposé au cours de l'utilisation de l'outil (2), ce calcul dépendant d'une valeur de vibration (A_set) indiquant la quantité de vibrations générée par l'outil (2) au cours de son fonctionnement et, d'une valeur de temps d'exposition (T) indiquant la durée pendant laquelle l'opérateur (3) est exposé aux vibrations.

2. Aspirateur (20) conforme à la revendication 1, dans lequel la valeur du temps d'exposition dérive d'un chronomètre (6) qui est mis en marche et arrêté automatiquement en fonction du signal électrique.

3. Aspirateur (20) conforme à la revendication 2, dans lequel le dispositif de contrôle (5) et le chronomètre (6) sont réalisés intégralement avec le boitier (21 a, 21 b) de l'aspirateur (20).

4. Aspirateur (20) conforme à l'une des revendications 1 à 3, dans lequel la valeur de vibrations est déterminée en ligne au cours du fonctionnement de l'outil (2) au moyen de moyens de détection des vibrations.

5. Aspirateur (20) conforme à l'une des revendications 1 à 3, dans lequel la valeur de vibrations est une valeur préréglée (A_set) assignée à l'outil (2) et fournie au dispositif de contrôle (5) avant le calcul du niveau de vibrations global.

6. Aspirateur (20) conforme à la revendication 5, dans lequel le dispositif de contrôle (5) comprend des moyens (8) permettant de régler la valeur de vibrations à une valeur (A_set) assignée à l'outil (2).

7. Aspirateur (20) conforme à la revendication 6, dans lequel les moyens (8) permettant de régler la valeur de vibrations comprennent des moyens de réglage manuels, en particulier un potentiomètre (8) ou un ou plusieurs boutons poussoirs (8a, 8b).

8. Aspirateur (20) conforme à la revendication 6, dans lequel la valeur de vibrations assignée à l'outil (2) est enregistrée dans l'outil (2) et les moyens (8) permettant de régler la valeur de vibrations comprennent des moyens de réception de la valeur de vibrations assignée à l'outil (2) enregistrée.

9. Aspirateur (20) conforme à la revendication 8, dans lequel les moyens de réception sont susceptibles de recevoir la valeur de vibrations assignée à l'outil (2) par une transmission sans fil, en particulier une transmission en radio fréquences.

10. Aspirateur (20) conforme à l'une des revendications 1 à 9, dans lequel l'aspirateur (20) est un dispositif mobile à commande électrique ou pneumatique.
